# EUROPEAN PATENT APPLICATION

(11) **EP 4 822 350 A1**
(43) Date of publication of application: **14.10.2026**
(21) Application number: 26159378.4
(22) Date of filing: 18.02.2026
(51) Int. Cl.: G01N 33/03

(54) **A DEVICE FOR DETECTING ADULTERATION IN OIL AND METHOD THEREOF**

(30) Priority: 09.04.2025 IN 202541034696
(71) Applicant: L & T Technology Services Limited, Chennai 600089 (IN)
(72) Inventor: MOHANACHAR, Yashoda, 577001 Davangere (IN); GOYAL, Kunal, 636007 Salem (IN); KOTHANDARAMAN, Mahendran, 635121 Krishnagiri (IN); PRAKASH, Akash Advaplar, 577513 Davangere (IN); MANAGULI, Sahana, 587314 Bagalkot (IN)
(74) Representative: Studio Torta S.p.A.

(57) **Abstract**

Disclosed herein is a device and method for detecting adulteration in oil. The device comprises a probe which is immersed into the oil. Further, the device comprises a plurality of sensors and a microcontroller coupled with each other. The plurality of sensors is configured to sense plurality of sensor data associated with the oil while the probe is in contact with the oil. Further, the microcontroller is configured to receive the plurality of sensor data from the plurality of sensors. Thereafter, the microcontroller is configured to process at least one of the plurality of sensor data to obtain one or more physical parameters corresponding to the oil. Finally, the microcontroller is configured to apply a pre-trained model on the at least one of the plurality of sensor data for detecting the adulteration in the oil in real-time based on the one or more physical parameters.

## Description

### TECHNICAL FIELD

The present invention pertains to the field of oil quality assessment and, more specifically, to a device and method for detecting adulteration in oil, particularly the presence of paraffin.

### BACKGROUND

The following description includes information that may be useful in understanding the present invention. It is not an admission that any of the information provided herein is prior art or relevant to the presently claimed invention, or that any publication specifically or implicitly referenced is prior art.

The adulteration of edible oils with paraffin oil presents significant public health and regulatory challenges. Paraffin oil, a hydrocarbon-based substance primarily intended for industrial applications such as lubrication, pharmaceutical formulations, and cosmetic products, lacks nutritional properties required for human consumption. Despite regulatory restrictions in many jurisdictions, paraffin oil is illicitly introduced into edible oils to increase volume and reduce production costs. Prolonged ingestion of paraffin-contaminated oil has been associated with adverse health effects, including gastrointestinal distress, liver and kidney damage, and an increased risk of cardiovascular diseases.

Conventional methods for detecting oil adulteration often rely on laboratory-based chemical analysis, which can be time-consuming, expensive, and inaccessible for routine field testing. The lack of efficient, real-time detection methods hinders regulatory enforcement and exposes consumers to potential health hazards. Given these limitations, there is a critical need for a rapid, cost-effective, and field-deployable solution capable of detecting adulteration in edible oils with high accuracy and sensitivity.

### SUMMARY OF THE DISCLOSURE

The present disclosure overcomes one or more shortcomings of the prior art and provides additional advantages discussed throughout the present disclosure. Additional features and advantages are realized through the techniques of the present disclosure. Other embodiments and aspects of the disclosure are described in detail herein and are considered a part of the claimed disclosure.

In one non-limiting embodiment of the present disclosure, a device for detecting adulteration in oil is disclosed. The device comprises a probe, a plurality of sensors configured to generate a plurality of sensor data associated with the oil and a microcontroller coupled to the probe and the plurality of sensors. Upon receiving the plurality of sensor data from the plurality of sensors while the probe is in contact with the oil, the microcontroller may be configured to process at least one of the plurality of sensor data to obtain one or more physical parameters corresponding to the oil. The microcontroller may be further configured to apply a pre-trained model on the at least one of the plurality of sensor data for detecting the adulteration in the oil in real-time based on the one or more physical parameters.

In another non-limiting embodiment of the present disclosure, the plurality of sensors may comprise an ultrasonic sensor, a temperature sensor, a capacitive sensor, an Infrared (IR) spectrometer sensor, a viscosity sensor, and a refractive index sensor. The plurality of sensor data comprise ultrasonic sensor data, temperature sensor data, capacitive sensor data, IR spectrometer sensor data, viscosity sensor data, and refractive index sensor data. The one or more physical parameters comprises a dielectric constant, a viscosity, absorption of IR light, and a refractive index of the oil.

In another non-limiting embodiment of the present disclosure, the model is trained by receiving first pre-stored sensor data, corresponding to a pure oil without presence of an adulterated component, from the plurality of sensors. Further, the model is trained by receiving second pre-stored sensor data and an actual value of an adulterated component concentration corresponding to an adulterated oil from the plurality of sensors and predicting concentration of the adulterated component in the adulterated oil by comparing the one or more physical parameters of the first pre-stored sensor data and the second pre-stored sensor data. Finally, the model is trained by iteratively training the model until an error between the predicted concentration of the adulterated component and the actual value of the adulterated component concentration is below a predefined error threshold, wherein the adulterated component comprises paraffin.

In another non-limiting embodiment of the present disclosure, wherein the ultrasonic sensor is configured to generate the ultrasonic sensor data comprising measurements used to determine level of the oil. The ultrasonic sensor is configured to emit an ultrasonic signal into the oil while the probe is in the contact with the oil. Further, the ultrasonic sensor is configured to calculate distance between the ultrasonic sensor and surface of the oil by measuring time taken for reception of the ultrasonic signal being reflected from the surface of the oil. Thereafter, the ultrasonic sensor is configured to determine the level of the oil based on the calculated distance. The level of the oil is used to determine volume of the oil.

In another non-limiting embodiment of the present disclosure, the temperature sensor is configured to generate the temperature sensor data comprising temperature of the oil and to compensate for temperature-dependent variations occurring in remaining sensor data of the plurality of sensor data based on the measured temperature sensor data.

In another non-limiting embodiment of the present disclosure, the refractive index sensor is configured to generate the refractive index sensor data by detecting variations in the refractive index of the oil. Further, the viscosity sensor is configured to generate the viscosity sensor data by detecting changes in the viscosity of the oil due to presence of the paraffin. Furthermore, the IR spectroscopy sensor is configured to generate the Infrared (IR) spectrometer sensor data by detecting chemical changes in the oil by emitting IR light through the oil, measuring an intensity of the emitted IR light through the oil and determining concentration of the paraffin based on absorption of the emitted IR light in the oil.

In another non-limiting embodiment of the present disclosure, the capacitive sensor is configured to generate the capacitive sensor data by calculating a capacitance by measuring the dielectric constant of the oil that varies due to the adulteration in the oil, and by determining presence of the adulterated component in the oil by measuring a capacitance difference between a baseline capacitance of the pure oil and the calculated capacitance of the oil under consideration. The baseline capacitance is calculated by measuring the dielectric constant of the pure oil without the presence of the adulterated component.

In another non-limiting embodiment of the present disclosure, the device further comprises a display unit configured to display labelled data comprising at least one of: the determined concentration of the paraffin, the temperature of the oil, and a percentage of the composition of the paraffin. The microcontroller is further configured to transmit the labelled data to an external device via a wireless communication protocol.

In another non-limiting embodiment of the present disclosure, the microcontroller is further configured to generate an alert if the determined concentration of the paraffin exceeds a predetermined paraffin concentration threshold. Moreover, the microcontroller is further configured to perform re-calibration of the plurality of sensor data if the IR spectrometer sensor data indicates that the determined concentration of the paraffin exceeds the predetermined paraffin concentration threshold without a corresponding change in the viscosity of the oil.

In another aspect of the present disclosure, a method of detecting adulteration in oil is disclosed. The method comprises receiving plurality of sensor data from a plurality of sensors while a probe is in contact with the oil. Thereafter, the method comprises processing at least one of the plurality of sensor data to obtain one or more physical parameters corresponding to the oil. Finally, the method comprises applying a pre-trained model on the at least one of the plurality of sensor data for detecting the adulteration in the oil in real-time based on one or more physical parameters.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF DRAWINGS

The embodiments of the disclosure itself, as well as a preferred mode of use, further objectives and advantages thereof, will best be understood by reference to the following detailed description of an illustrative embodiment when read in conjunction with the accompanying drawings. One or more embodiments are now described, by way of example only, with reference to the accompanying drawings in which:
**Fig. 1a** illustrates an exemplary device for detecting adulteration in oil, in accordance with an embodiment of the present disclosure.
**Fig. 1b** illustrates an exemplary detailed view of components of the device for detecting adulteration in oil, in accordance with an embodiment of the present disclosure.
**Fig. 2** illustrates an exemplary process flow diagram for detecting adulteration in oil, in accordance with an embodiment of the present disclosure.
**Fig. 3** depicts a flow diagram of a method for detecting adulteration in oil, in accordance with an embodiment of the present disclosure.

The figures depict embodiments of the disclosure for purposes of illustration only. One skilled in the art will readily recognize from the following description that alternative embodiments of the structures and methods illustrated herein may be employed without departing from the principles of the disclosure described herein.

### DETAILED DESCRIPTION

The foregoing has broadly outlined the features and technical advantages of the present disclosure in order that the detailed description of the disclosure that follows may be better understood. It should be appreciated by those skilled in the art that the conception and specific embodiment disclosed may be readily utilized as a basis for modifying or designing other structures for carrying out the same purposes of the present disclosure.

Various embodiments of the present invention will now be described more fully hereinafter with reference to the accompanying drawings, in which some, but not all embodiments of the invention are shown. Indeed, the invention may be embodied in many different forms and should not be construed as limited to the embodiments set forth herein. Rather, these embodiments are provided so that this disclosure will satisfy applicable legal requirements.

The term "or" is used herein in both the alternative and conjunctive sense, unless otherwise indicated.

The terms "illustrative," "example," and "exemplary" are used to be examples with no indication of quality level. Like numbers refer to like elements throughout.

The phrases "in an embodiment," "in one embodiment," "according to one embodiment," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The word "exemplary" is used herein to mean "serving as an example, instance, or illustration." Any implementation described herein as "exemplary" is not necessarily to be construed as preferred or advantageous over other implementations.

If the specification states a component or feature "can," "may," "could," "should," "would," "preferably," "possibly," "typically," "optionally," "for example," "often," or "might" (or other such language) be included or have a characteristic, that particular component or feature is not required to be included or to have the characteristic. Such components or features may be optionally included in some embodiments, or it may be excluded.

The phrase "artificial intelligence" refers to the field of studying artificial intelligence or methodology for making artificial intelligence, and machine learning refers to the field of defining various issues dealt with in the field of artificial intelligence and studying methodology for solving the various issues. Machine learning is defined as an algorithm that enhances the performance of a certain task through a steady experience with the certain task.

Turning now to the drawings, the detailed description set forth below in connection with the appended drawings is intended as a description of various configurations and is not intended to represent the only configurations in which the concepts described herein may be practiced. The detailed description includes specific details for the purpose of providing a thorough understanding of various concepts with like numerals denote like components throughout the several views. However, it will be apparent to those skilled in the art that these concepts may be practiced without these specific details.

To overcome the challenges presented by the existing methods, the present invention seeks to provide a novel approach for detecting adulteration in oil. In particular, the present disclosure aims to provide a compact, handheld, and battery powered device and method for detecting adulteration in oil. The device may serve as an efficient, accurate, and reliable means of identifying impurities, or substances mixed with oil, ensuring quality control and compliance with industry standards. Further, the proposed device and method may be applied across various types of oils, including edible oils, enhancing safety, performance, and consumer confidence. A detailed explanation of oil adulteration detection technique is provided in forthcoming paragraphs.

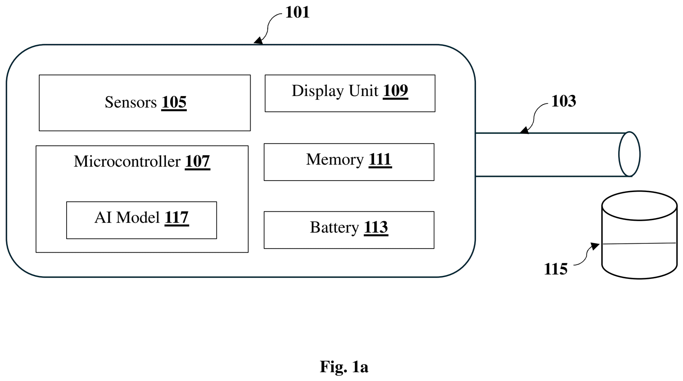

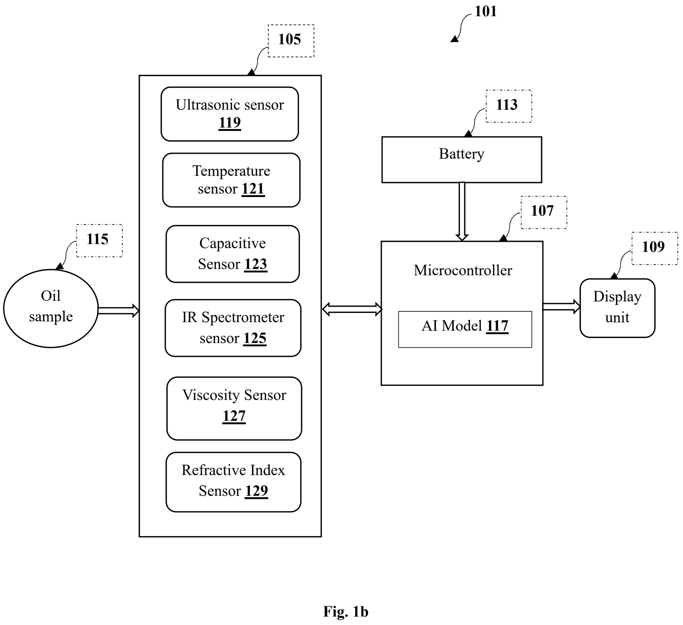

**Fig. 1a** illustrates an exemplary device 101 and its constituent elements for detecting adulteration in oil 115 (interchangeably referred to as oil sample), in accordance with an embodiment of the present disclosure. The exemplary device 101 may be configured as a standalone computing device 101 designed for the detection of adulterants in oil, intended for use by end consumers. All the constituent elements depicted in Fig. 1 are essential for the operation of the device 101, however, the device 101 may further comprise additional elements beyond those explicitly illustrated. The device 101 may include various functional components, such as a probe 103, a plurality of sensors 105, a microcontroller 107, a display unit 109, a memory 111, and a power source 113 such as a battery. These components may be operatively coupled to facilitate the detection of the adulteration in the oil 115 through sensor-based analysis, data processing, and real-time determination of adulterated component (also referred to as 'paraffin') concentration. In some embodiments, the battery 113 may serve as a primary power source, enabling the operation of the plurality of sensors 105, the microcontroller 107, the display unit 109, and other electronic components. The battery 113 may be configured to supply a stable voltage and current to ensure uninterrupted functionality of the device 101.

In some embodiments, the probe 103 may be configured as an elongated, cylindrical structure for easy immersion into oil containers, while in other embodiments, it may be designed as a planar or modular structure for integration into automated testing systems. Suitable materials for the probe 103 may include, but are not limited to, stainless steel, food-grade polymers, ceramics, or composite materials exhibiting high thermal and chemical stability. It may be well appreciated to a person skilled in the art that the structural configuration of the probe 103 may vary depending on the specific application requirements and detection methodology employed by the device 101.

In some embodiments, the probe 103, as incorporated within the exemplary device 101 illustrated in Fig. 1a, may serve as a primary interface for interaction with the oil sample 115 and is a critical constituent element of the device 101. The probe 103 may be structurally configured to facilitate optimal contact with the oil sample 115 while ensuring compatibility with various types of oils, including but not limited to edible oils. Thus, the probe 103 may be immersed in the oil 115 to detect whether the oil 115 is adulterated or not.

Further, the plurality of sensors 105 may include, but is not limited to, an ultrasonic sensor 119, a temperature sensor 121, a capacitive sensor 123, an infrared (IR) spectrometer sensor 125, a viscosity sensor 127, and a refractive index sensor 129. It is to be understood that the device 101 may not be limited to the aforementioned sensors and may incorporate additional sensors as required for detecting adulteration in oil. The selection and configuration of the plurality of sensors 105 may be adapted based on the specific parameters to be analysed, such as the type of the oil 115 under examination. The plurality of sensors 105 may be interlinked with each other while performing adulteration detection in the oil. The plurality of sensors 105 may be configured to provide plurality of sensor data to the microcontroller 107 while the probe 103 is in contact with the oil.

In one implementation, the microcontroller 107 may be implemented as one or more processors, microprocessors, microcomputers, digital signal processors, central processing units, state machines, logic circuitries, and/or any devices that manipulate signals based on operational instructions. Among other capabilities, the microcontroller 107 may be configured to fetch and execute computer-readable instructions stored in the memory 111. In some embodiments, the microcontroller 107 may be configured to implement the various functionalities of the device 101. The microcontroller 107 may be communicatively coupled to the plurality of sensors 105, and the display 109 for implementing the functions of the system 101. Further, the microcontroller 107 may comprise an Artificial Intelligence (AI) model 117 for determining presence and concentration of the adulterated component in the oil. The microcontroller 107 may be configured to receive the plurality of sensor data (e.g., raw sensor data in the form of analog signals) from the plurality of sensors 105. Thereafter, the microcontroller 107 may process the plurality of sensor data to determine the adulteration in the oil.

In some embodiments, the display unit 109 may be an output device for displaying the determined concentration of the adulterated component in visual or tactile form on the device 101 to the user or an operator. In an example, the display may include but not limited to a Liquid Crystal Display (LCD), Organic LED (OLED), miniLED, microLED, In-plane Switching LCD (IPS-LCD), Active-Matrix organic Light-Emitting Diode (AMOLED).

In one implementation, the AI model 117 (interchangeably referred to as model) may be implemented as one or more processors, microprocessors, microcomputers, microcontrollers, digital signal processors, central processing units, state machines, logic circuitries, and/or any devices that manipulate signals based on operational instructions. The model 117 may serve as a pre-trained model utilized for determining adulteration in oil 115 in a real time. In one implementation, the model 117 may be internally configured within the microcontroller 107. Alternatively, the model 117 may be externally configured or separately built outside the microcontroller 107. In an exemplary embodiment, the model 117 may be stored in the memory 111 and the microcontroller 107 may fetch the instructions from the memory 111 to perform the desired functions. Further, a detailed explanation of training the model 117 is explained in forthcoming paragraphs.

In some embodiments, the memory 111 may include one or more non-transitory computer-readable storage media, such as RAM, ROM, EEPROM, EPROM, one or more memory devices, flash memory devices, etc., and combinations thereof. In an embodiment, the memory 111 may comprise the plurality of sensor data and other data. The sensor data may comprise, but not limited to, ultrasonic sensor data, temperature sensor data, capacitive sensor data, IR spectrometer sensor data, viscosity sensor data, and refractive index sensor data. The plurality of sensor data may be interdependent for detecting adulteration in oil as explained in upcoming paragraphs. In some embodiments, the plurality of sensor data may be pre-available sensor data that is utilized for training the model 117. It may be appreciated by a person skilled in the art that the plurality of sensor data may be stored within the memory 111 in the form of various data structures. Further, the memory 111 may also store other data such as temporary data and temporary files, generated by the microcontroller 107 for performing the various functions of the present disclosure. A detailed explanation of the processing for determining the adulteration in the oil 115 is explained in reference to Fig. 1b.

**Fig. 1b** illustrates an exemplary detailed view of the components of the device 101 for detecting adulteration in oil, in accordance with an embodiment of the present disclosure. In operation, when the probe 103 is in contact with the oil sample 115, the ultrasonic sensor 119 may be configured to generate ultrasonic sensor data comprising measurements utilized for determining the level of the oil. The ultrasonic sensor 119 may be configured to measure distance to the oil surface by emitting an ultrasonic signal into the oil 115 while the probe 103 remains in contact with the oil 115. Thereafter, the ultrasonic sensor 119 may be configured to determine time taken corresponding to the reception of the ultrasonic signal reflected from the oil surface. Based on the measured time taken, the ultrasonic sensor 119 may calculate the distance to the oil surface using the following equation: $Distance \left(D\right) = \left(ultrasonic signal duration \left(microsecond\right)*speed of sound \left(m/s\right)\right) / 2$ (1) Once the distance is measured, the level (i.e., height) of the oil 115 may be obtained using the following equation: $Oil level \left(h\right) = total height of the oil container \left(H\right) - measured distance \left(D\right)$ (2) For example, in an embodiment where the total height (H) of the cylindrical container is 100 cm and the ultrasonic sensor 119 measures the distance (D) of 40cm from the ultrasonic sensor 119 to the oil surface, the oil level (h) may be calculated as: $h = 100 - 40 = 60 cm$ Accordingly, the oil level may be determined to be 60 cm from the bottom of the oil container. Further, the level of the oil 115 may be subsequently utilized to determine volume of the oil 115 using the below equation: $Volume \left(V\right) = area \left(A\right) * h$ (3) Where: "V" may be referred to as volume of the oil, "A" may be referred to as a cross-sectional area of oil container, and "h" may be referred to as the determined level (height (h)) of the oil. In an exemplary embodiment, where the container is a rectangular tank with a known length, width, and measured height, the volume of oil 115 may be determined using the equation: $V = L * W * h$ (4) where "L" may represent the length of the container, "W" may represent the width, and "h" may represent the level of the oil. Assuming, a rectangular tank having a length of 100 cm, a width of 50 cm, and the measured oil level of 60 cm, the volume of oil 115 may be calculated as 300,000 cm³, which corresponds to 300 liters. It may be well appreciated by a person skilled in the art that the above example is provided for better clarity purposed and should not be taken into liming sense.

In certain embodiments, if the determined oil level is below a predefined oil level threshold, the oil 115 may be deemed unsuitable for further analysis and may be discarded. In such instances, the oil 115 may not be preferred for detecting adulteration and may be referred to as oil level not detected. Conversely, if the determined oil level exceeds the predefined oil level threshold, remaining sensors of the plurality of sensors 105 may be activated to analyse sensor data associated with the oil for detecting the adulteration or other qualitative assessments of the oil. Accordingly, while the ultrasonic sensor 119 may not directly influence the determination of paraffin concentration in the oil, it plays a critical role in ensuring the accuracy and consistency of sensor data by precisely measuring the level of the oil. The determination of the oil level enables proper calibration and normalization of the sensor data obtained from the plurality of sensors 105, thereby enhancing the reliability of adulteration detection.

In certain embodiments, the temperature sensor 121 may be configured to generate temperature sensor data by measuring the temperature of the oil 115 using the below equation: $Temperature \left(°C\right) = \left(Vout*1000\right) / 10$ (5) Where "Vout" may be referred to as temperature sensor output voltage (in volts). In an exemplary embodiment, assuming that the temperature sensor 121 outputs 0.25V:
Temperature = 0.25*1000/10 = 25°C. Thus, the measured temperature is 25°C. It may be well appreciated by a person skilled in the art that the above example is provided for better clarity purposed and should not be taken into liming sense.

In certain embodiments, the temperature sensor data generated by the temperature sensor 121 may be utilized to compensate for temperature-dependent variations in the sensor data obtained from the remaining plurality of sensors 105. In some embodiments, the measured temperature may directly influence viscosity and refractive index of the oil. Further, temperature compensation may be essential for adjusting the IR spectrometer sensor data, as the solubility of paraffin and its spectral characteristics may exhibit temperature-dependent variations. Thus, the temperature sensor data may be employed to enhance the accuracy and reliability of oil adulteration analysis by mitigating temperature-induced discrepancies in sensor measurements.

In certain embodiments, the capacitive sensor 123 may be configured to generate capacitive sensor data by calculating the capacitance of the oil sample 115. The capacitance of the oil sample 115 may be calculated based on the measurement of its dielectric constant which may vary due to the presence of adulterated component in the oil. Further, the capacitive sensor 123 may be configured to determine the presence of the adulterated component in the oil 115 by measuring a capacitance difference between a baseline capacitance of a pure oil (having no presence of paraffin oil or any impurity) and the calculated capacitance of the oil 115 under consideration. The capacitance difference may be calculated using the below equation: $Capacitance difference \left(C\right) = Csample - Cbaseline$ (6) Where "Csample" may be referred to as capacitance of the oil sample 115 and "Cbaseline" may be referred to as capacitance of the pure oil. The baseline capacitance may be calculated by measuring the dielectric constant of the pure oil. The pure oil may be referred to as an oil without the presence of the adulterated component or impurities. Hence, the capacitance difference between the calculated capacitance of the oil 115 under consideration and the baseline capacitance of the pure oil may indicate the presence or absence of the adulteration, thereby facilitating the identification of impurities or substances mixed with the oil. Accordingly, the calculated capacitance of the oil 115 may serve as an indicator of variations in its dielectric constant, which may fluctuate in response to changes in the paraffin concentration within the oil. A higher or lower paraffin concentration may result in a corresponding shift in the dielectric properties of the oil, thereby affecting the measured capacitance.

In certain embodiments, the capacitance sensor data obtained from the capacitive sensor 123 may be influenced by additional factors, including the viscosity sensor data and refractive index sensor data of the oil. Furthermore, temperature variations may also indirectly affect the dielectric constant of the oil, thereby introducing temperature-dependent changes in the measured capacitance of the oil. Accordingly, temperature compensation may be applied to enhance the accuracy and reliability of oil adulteration detection.

In certain embodiments, the IR spectrometer sensor 125 may be configured to generate IR spectrometer sensor data by detecting chemical variations in the oil. The IR spectrometer sensor 125 may be configured to emit infrared (IR) light through the oil sample 115 and analyze the interaction between the emitted IR light and the chemical composition of the oil 115. Upon emission, the IR spectrometer sensor 125 may be further configured to measure the intensity of the transmitted or absorbed IR light after interaction with the oil. The measured IR spectrometer sensor data may be processed using a predefined equation reproduced below to quantify chemical changes in the oil, facilitating the identification of adulterated component: $A = - log \left(I/I0\right)$ (7) where, "A" is absorbance, "I0" is initial intensity, and "I" is measured intensity. Accordingly, the IR spectrometer sensor data may facilitate chemical detection by analyzing the molecular absorption characteristics of IR light to determine the paraffin concentration in the oil 115. In particular, the IR spectrometer sensor 125 may be configured to measure the spectral absorption or transmission of the oil 115 at various wavelengths. Further, the absorption pattern may directly correlate with the chemical composition of the oil 115, including the presence and concentration of paraffin.

In certain embodiments, the temperature sensor data, viscosity sensor data, and refractive index sensor data may influence the spectral properties of the IR spectrometer sensor data. Variations in temperature, viscosity, and refractive index may introduce spectral shifts or alterations in absorption characteristics, potentially affecting the accuracy of paraffin concentration measurements. Therefore, the temperature sensor data, viscosity sensor data, and refractive index sensor data may be utilized for calibrating and correcting the IR spectrometer sensor data to ensure precise chemical analysis of the oil sample 115.

In some embodiments, the refractive index sensor 129 may be configured to measure refractive index sensor data for detecting variations in the refractive index of the oil 115. In other words, the refractive index sensor 129 may be configured to determine the extent to which light bends as it passes through the oil 115, thereby providing an indication of the oil's composition and potential adulteration. In an exemplary embodiment, the refractive index sensor data may be determined using the following predefined equation, where the refractive index may be computed based on the interaction of light with the oil medium: $I = I 0 {e}^{∧} \left(-ax\right)$ (8) Where 'I0' may be referred to as initial intensity of the IR light, 'I' may be referred to as emitted intensity, 'a' may be referred to as absorption coefficient and 'x' may be referred to as thickness of the oil sample 115. Thus, the measured refractive index may serve as a parameter for assessing the purity and quality of the oil 115 by detecting deviations from a reference refractive index value corresponding to the pure oil. For example, considering the refractive index sensor may detect adulteration in an olive oil. Pure olive oil may have known reference refractive index value of approximately 1.467 to 1.470 at 20°C. The refractive index sensor may measure the refractive index of the oil 115 and may compare the measured refractive index value to the above-mentioned reference value. Suppose a sample of olive oil is tested, and the sensor detects a refractive index of 1.475, which is higher than the expected range. This deviation suggests possible adulteration, potentially due to the presence of paraffin oil, which has a higher refractive index (typically 1.480 - 1.500). Accordingly, the refractive index sensor 129 may be configured to measure variations in the refractive index of the oil 115, which may change as the paraffin concentration increases. The measured refractive index data may provide an indication of alterations in the oil's composition, thereby facilitating the detection of adulterated component such as paraffin.

Furthermore, the refractive index of the oil 115 may be dependent on both temperature and paraffin concentration, as variations in these parameters may alter the optical properties of the oil. Accordingly, the refractive index sensor 129 may be configured to operate in conjunction with the temperature sensor 121 and the IR spectrometer sensor 125 to compensate for temperature-induced fluctuations and spectral variations. The combined temperature sensor data and the IR spectrometer sensor data may enhance the accuracy of refractive index measurements, thereby improving the reliability of oil adulteration detection and analysis.

In some embodiments, the viscosity sensor 127 may be configured to generate viscosity sensor data by detecting changes in the viscosity of the oil 115 due to the presence of the paraffin. The viscosity of the oil 115 may be determined using the following equation: $Viscosity = Vout * calibration factor$ Where "Vout" represents the viscosity sensor output voltage, and the calibration factor may be predefined based on empirical measurements. In other words, the viscosity sensor 127 may be configured to measure the oil's resistance to flow, which serves as a critical indicator of paraffin presence within the oil sample 115. A higher paraffin concentration may increase the oil's viscosity, thereby altering its flow characteristics.

In some embodiments, the viscosity of the oil 115 may also be influenced by temperature variations, as changes in temperature may alter the fluidity of the oil. Additionally, the IR spectrometer sensor data may provide insights into the presence of long-chain hydrocarbons, which contribute to an increase in the viscosity of the oil 115. Accordingly, the integration of viscosity sensor data, temperature sensor data, and IR spectrometer sensor data may facilitate a comprehensive assessment of oil adulteration and ensure accurate detection of the adulterated component in the oil 115.

Once the plurality of sensor data is generated as explained above, the microcontroller 107 may be configured to receive the plurality of sensor data from the plurality of sensors 105. The microcontroller 107 may be configured to process the received plurality of sensor data which may be initially in the form of analog signals. The microcontroller 107 may be configured to convert the received analog signals into digital signals or digital data using Analog-to-Digital Converter (ADC) pins.

In some embodiments, the plurality of sensor data may comprise undesirable variations or distortions (referred to as 'noise') that may affect the accuracy and reliability of sensor measurements. Various factors which may contribute to noise in the plurality of sensor data may include, but not limited to, electrical interference, thermal fluctuations, power supply variations, mechanical vibrations, external disturbances, sensor aging, and drift, etc., These factors may lead to inconsistencies in the sensor data, thereby impacting the precision of oil adulteration detection. Therefore, to enhance the accuracy and reliability of plurality of sensor data, the microcontroller 107 may implement noise filtering techniques. These noise filtering techniques may include, but are not limited to, moving average filters, low-pass filters, median filters, and other digital signal processing (DSP) algorithms. Such filtering mechanisms may be applied to eliminate undesirable variations and fluctuations in the plurality of sensor data, ensuring more stable and accurate measurements for further processing and analysis.

Subsequently, the microcontroller 107 may be configured to adjust the viscosity sensor data, refractive index sensor data, and capacitive sensor data based on the temperature sensor data. Since temperature variations may directly affect the viscosity, refractive index, and capacitance of the oil 115, the microcontroller 107 may apply compensation techniques to normalize the plurality of sensor data or sensor outputs. Such compensation techniques may include temperature correction techniques or calibration models that account for temperature-induced variations in the plurality of sensor data. By implementing these compensation techniques, the device 101 may ensure enhanced accuracy and reliability in detecting adulteration in the oil 115, thereby improving the overall performance of the oil quality assessment process.

Additionally, in some embodiments, the microcontroller 107 may be configured to generate an error message if the determined oil level falls below a predetermined oil level threshold. If the oil level is insufficient for accurate measurement, the microcontroller 107 may prevent further processing of the sensor data and may notify the user regarding the insufficient oil level. This feature ensures that the device operates under optimal conditions, preventing erroneous readings in the plurality of sensor data and ensuring the reliability of oil adulteration detection.

Further, the microcontroller 107 may be configured to convert the raw analog signal values of the plurality of sensor data into one or more meaningful physical parameters, including, but not limited to, viscosity (measured in centipoise), refractive index, and dielectric constant. The conversion process may utilize pre-programmed calibration curves, lookup tables, or mathematical models to map the raw analog signal values to corresponding physical properties or parameters of the oil 115. Additionally, the microcontroller 107 may be further configured to analyze the IR spectrometer sensor data to identify paraffin-related spectral peaks. The identification of these spectral peaks may enable the detection and quantification of paraffin concentration in the oil 115, facilitating an accurate assessment of potential adulteration.

Upon completion of processing, the microcontroller 107 may be configured to aggregate the plurality of sensor data into a single training dataset. The microcontroller 107 may then apply a pre-trained model 117 to the plurality of sensor data to enable real-time detection of the adulteration in the oil 115 which is under consideration. Furthermore, the training dataset may be utilized to train the AI model 117 for determining paraffin concentration based on one or more physical parameters derived from the plurality of sensor data. In certain embodiments, the AI model 117 may be internally implemented within the microcontroller 107, allowing for on-device processing and real-time analysis. Alternatively, in some embodiments, the AI model 117 may be externally hosted, such as on a cloud-based computing platform. In such cases, the microcontroller 107 may be configured to communicate with the externally hosted AI model 117 via a wireless communication network or other communication means to facilitate real-time adulteration detection in the oil 115.

In some embodiments, the AI model 117 may be configured to undergo training by receiving first pre-stored sensor data from the plurality of sensors 105. The first pre-stored sensor data may correspond to the pure oil, where no adulterated component such as paraffin is present. In some embodiments, the first sensor data may be stored in the memory 111. Subsequently, the AI model 117 may receive second pre-stored sensor data along with a corresponding actual value of an adulterated component concentration in the adulterated oil. As described above, the adulterated oil may comprise paraffin concentration in the oil. The AI model 117 may then predict the concentration of the adulterated component in the adulterated oil by analyzing and comparing the one or more physical parameters derived from the first pre-stored sensor data (associated with the pure oil) and the second pre-stored sensor data (associated with the adulterated oil). Further, the AI model 117 may be iteratively trained by adjusting its parameters until an error between the predicted concentration of the adulterated component and the actual value of the adulterated component concentration is below a predefined error threshold. This iterative training process enhances the accuracy and reliability of the AI model 117 in detecting paraffin adulteration. In some embodiments, the predefined error may be established based on or more statistical metrics including, but not limited to, an absolute error, a Mean Squared Error, a relative percentage error, a coefficient of determination, etc., For example, if the actual concentration of an adulterant in oil is 5.0%, the AI model's prediction must be within 4.5% to 5.5% (assuming an absolute error threshold of **0.5%**) to be considered accurate. If the predicted value falls outside this range, then the training process continues with further adjustments to the AI model parameters. By implementing this predefined error threshold, the AI model may ensure reliable and precise detection of oil adulteration, reducing false positives and negatives while improving overall device performance.

In some embodiments, the AI model may be trained using various types of cooling oils. Once the training is completed, the AI model may be utilized to detect adulteration in oil in real-time.

In some embodiments, the present disclosure may further implement one or more suitable computational techniques, including but not limited to, weighted averaging, predictive algorithms, and regression-based models, for determining the paraffin concentration in the oil 115. Thus, the present disclosure employs a dual-step approach, combining both a sensor-based detection approach and an AI model-based analytical approach, to effectively determine both the presence and concentration of paraffin or any other adulterated component in the oil 115.

Once the concentration of the adulterated component is determined, the microcontroller 107 may be configured to validate the determined concentration of the adulterated component by analyzing the consistency of IR spectrometer sensor data of the oil 115 under consideration, the one or more physical parameters of the oil 115 under consideration, and the baseline capacitance of the pure oil. This validation process ensures accuracy and reliability in detecting adulteration within the oil sample 115. Upon successful validation, the microcontroller 107 may be configured to generate and display a first notification on the display unit 109, indicating that the oil 115 is adulterated. Additionally, the microcontroller 107 may be configured to display labelled data associated with the adulteration analysis. The labelled data may include, but is not limited to, the determined concentration of paraffin, the temperature of the oil 115, and the percentage composition of paraffin in the oil 115. Furthermore, the microcontroller 107 may be configured to transmit the labelled data to an external device via a wireless communication protocol, such as Bluetooth, enabling remote monitoring and analysis of the oil adulteration detection results. In some embodiments, the external device may be referred to as any remote system capable of receiving, processing and analyzing the labelled data transmitted by the microcontroller 107. In some embodiments, the external device may be managed by different entities including, but not limited to, researchers or scientists, or end users, industrial operators, etc., depending on the application and use case of the oil adulteration device.

In some embodiments, the microcontroller 107 may be configured to generate an alert upon determining that the concentration of the paraffin in the oil 115 exceeds a predetermined paraffin concentration threshold. The alert may be provided to the user in at least one of an audio form, or a visual form, haptic feedback, or a combination thereof. Alternatively, if the determined concentration of the paraffin in the oil 115 is below the predetermined paraffin concentration threshold, then the microcontroller 107 may be configured to generate and display a second notification message on the display unit 109, indicating that the oil 115 may be classified as non-adulterated. In some embodiments, the microcontroller 107 may be configured to perform re-calibration of the plurality of sensor data upon detecting an inconsistency between the IR spectrometer sensor data and the viscosity sensor data. Specifically, if the IR spectrometer sensor data indicates that the determined paraffin concentration exceeds the predetermined paraffin concentration threshold without a corresponding change in the viscosity of the oil, the microcontroller 107 may initiate a recalibration process to enhance the accuracy of the sensor measurements. Thus, the recalibration process may be required scenario to enhance the accuracy of the plurality of sensor data generated by the plurality of sensors because the detected paraffin concentration may be inconsistent with the expected physical behaviour or parameters of the oil 115.

Accordingly, the present disclosure provides a compact, battery-operated, reusable, and field-deployable device 101 configured to integrate the plurality of sensors 105 which are interdependent for detecting adulteration in oil. The device 101 is designed to enable real-time determination of adulterated components in oil, thereby facilitating immediate analysis and decision-making for the user. The disclosed device 101 and method offer a rapid, cost-effective, and user-friendly solution for detecting oil 115 adulteration. In particular, the present disclosure provides an efficient, accurate, and reliable mechanism for identifying impurities or substances mixed with oil, thereby ensuring quality control and regulatory compliance in accordance with industry standards. Furthermore, the disclosed device 101 and method may be applicable across various categories of oils, including but not limited to edible oils, thereby enhancing safety, performance, and consumer confidence in oil quality.

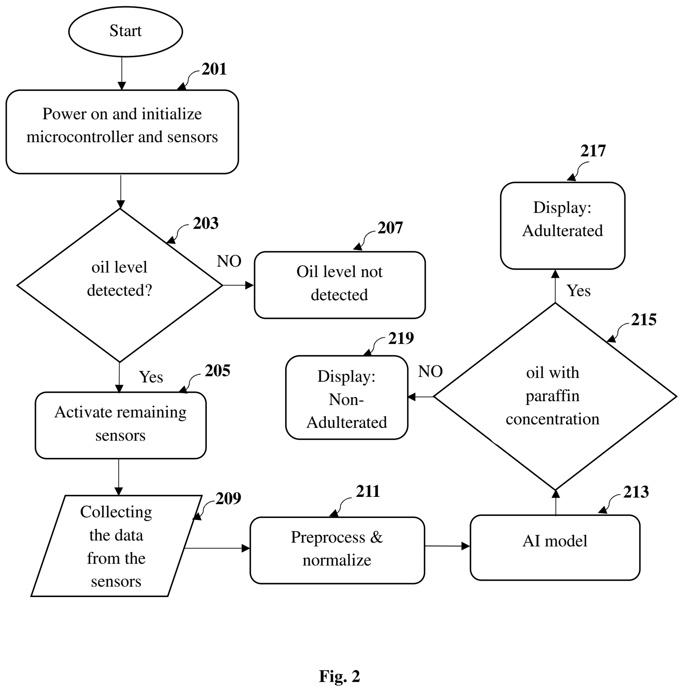

Moving towards Fig. 2 that illustrates an exemplary process flow diagram for detecting adulteration in oil, in accordance with an embodiment of the present disclosure.

According to an embodiment of the present disclosure, at step 201, the process for determining the adulteration in the oil 115 may be initiated, wherein power is provided to initialize the microcontroller 107 and the ultrasonic sensor 119. Further, at step 203, the process may determine the level of the oil 115 using the ultrasonic sensor 119 by emitting ultrasonic signals towards the oil surface and calculating the distance between the ultrasonic sensor 119 and the surface of the oil 115 by measuring the time taken by the emitted ultrasonic signal to reflect from the oil surface. Thereafter, the oil level may be calculated based on the determined distance and the known total height of the oil container. Upon detecting the level of the oil 115, the process may proceed to step 205, for triggering activation of remaining sensors of the plurality of sensors 105 including the temperature sensor 121, the capacitive sensor 123, the IR spectrometer sensor 125, the refractive index sensor 129, and the viscosity sensor 127. Each of these sensors may be configured to generate corresponding sensor data associated with the oil as explained in the above paragraphs of the specification. Alternatively, as indicated in step 207, if the level of the oil 115 is not detected, further analysis may not be performed and the oil 115 may be discarded.

Following activation, the process may involve collecting the plurality of sensor data from the plurality of sensors 105 as indicated in step 209. Further, at step 211, the plurality of sensor data may be processed by the microcontroller 107. The raw sensor data typically in the form of analog signals may be converted into digital format using ADC pins. Additionally, normalization may be performed on the plurality of sensor data to remove redundancies and enhance the accuracy of subsequent analysis. Thereafter, the processed sensor data may be provided to the AI model 117 as shown in step 213 for determining the concentration of the adulterated component in the oil 115. Further, at step 215, the determined concentration of the adulterated component may be compared with the predetermined paraffin concentration threshold. Upon determining that the concentration of the adulterated component exceeds the predetermined adulterated concentration threshold, the oil 115 may be classified as adulterated oil as shown in step 217. Alternatively, upon determining that the concentration of the adulterated component is below the predetermined adulterated concentration threshold, the oil 115 may be classified as non-adulterated oil as shown in step 219.

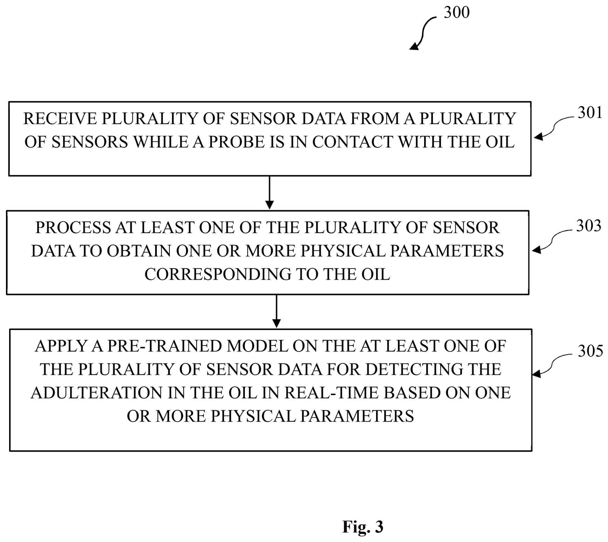

Moving towards Fig. 3 shows steps of a method 300 for detecting adulteration in oil, in accordance with an embodiment of the present disclosure. The method starts at step 301, which may comprise receiving plurality of sensor data from the plurality of sensors 105 while the probe 103 is in contact with the oil. In an embodiment, the plurality of sensors 105 may comprise the ultrasonic sensor 119, the temperature sensor 121, the capacitive sensor 123, the Infrared (IR) spectrometer sensor 125, the viscosity sensor 127, and the refractive index sensor 129. Further, the plurality of sensor data may comprise ultrasonic sensor data, temperature sensor data, capacitive sensor data, IR spectrometer sensor data, viscosity sensor data, and refractive index sensor data.

In some embodiments, the method may comprise generating the ultrasonic sensor data using the ultrasonic sensor 119. The ultrasonic sensor data may comprise measurements used for determining the level of the oil 115 by emitting an ultrasonic signal into the oil 115 while the probe 103 is in contact with the oil. Further, the method may comprise calculating distance between the ultrasonic sensor 119 and the surface of the oil 115 by measuring the time taken while receiving the ultrasonic signal being reflected from the oil surface. Thereafter, the method may comprise determining the level of the oil 115 based on the calculated distance. Finally, the level of the oil 115 may be used to determine volume of the oil.

Once the level of the oil 115 is detected, the method may proceed further to activate the other sensors and generate sensor data corresponding to the other sensors. In particular, the method may comprise generating the temperature sensor data comprising the temperature of the oil 115 and compensating for temperature-dependent variations occurring in the other sensor data of the plurality of sensor data based on the measured temperature sensor data. The method may measure the temperature sensor data using the temperature sensor 121.

In some embodiments, the method may comprise generating the refractive index sensor data to detect variations in the refractive index of the oil 115 using the refractive index sensor 129. Further, the method may comprise generating the viscosity sensor data by detecting changes in the viscosity of the oil 115 due to the presence of the paraffin or the adulterated component. The method may generate the viscosity sensor data using the viscosity sensor 127.

In some embodiments, the method may comprise generating the IR spectrometer sensor data by detecting chemical changes in the oil 115 by emitting IR light into the oil. Further, the method may comprise measuring the intensity of the emitted IR light through the oil 115 and determining concentration of the paraffin based on the absorption of the emitted IR light in the oil.

In some embodiments, the method may comprise generating the capacitive sensor data by calculating the capacitance. The capacitance may be calculated based on dielectric constant that may vary due to the adulteration in the oil. Furthermore, the method may comprise determining presence of the adulterated component in the oil 115 by measuring a capacitance difference between a baseline capacitance of the pure oil and the calculated capacitance of the oil 115 under consideration. The baseline capacitance may be calculated based on the dielectric constant of the pure oil without the presence of the adulterated component.

At step 303, the method 300 may include processing at least one of the plurality of sensor data to obtain one or more physical parameters corresponding to the oil. In some embodiments, the one or more physical parameters may comprise a dielectric constant, a viscosity in centipoise, absorption of IR light at specific wavelengths, and a refractive index of the oil.

At step 305, the method 300 may include applying the pre-trained model 117 on the at least one of the plurality of sensor data for detecting the adulteration in the oil 115 in real-time based on one or more physical parameters. In some embodiments, the method may comprise training the model 117 by receiving first pre-stored sensor data corresponding to a pure oil without the presence of an adulterated component. Thereafter, the method may comprise receiving second pre-stored sensor data and an actual value of an adulterated component concentration corresponding to an adulterated oil. The first pre-stored sensor data and the second pre-stored sensor data may be received from the plurality of sensors 105. Further, the method may comprise predicting concentration of the adulterated component in the adulterated oil by comparing the one or more physical parameters of the first pre-stored sensor data and the second pre-stored sensor data. Finally, the method may comprise iteratively training the model 117 until an error between the predicted concentration of the adulterated component and the actual value of the adulterated component concentration is below a predefined error threshold.

In some embodiments, the method may comprise displaying labelled data on the display unit 109 to the user. The labelled data may comprise at least one of the determined concentration of the paraffin, the temperature of the oil, and a percentage of the composition of the paraffin. In some embodiments, the method may comprise transmitting the labelled data to an external device via a wireless communication protocol.

In some embodiments, the method may comprise generating an alert if the determined concentration of the paraffin exceeds a predetermined paraffin concentration threshold. Further, the method may comprise perform re-calibration of the plurality of sensor data if the IR spectrometer sensor data indicates that the determined concentration of the paraffin exceeds the predetermined paraffin concentration threshold without a corresponding change in the viscosity of the oil.

The foregoing method descriptions and the process flow diagrams are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," etc. are not intended to limit the order of the steps; these words are simply used to guide the reader through the description of the methods. Further, any reference to claim elements in the singular, for example, using the articles "a," "an" or "the" is not to be construed as limiting the element to the singular.

As used herein, the term unit may be implemented in hardware and/or in software. If the unit is implemented in hardware, the unit may be configured as a device, e.g., as a computer or as a processor or as a part of a system, e.g., a computer system. If the unit is implemented in software, the unit may be configured as a computer program product, as a function, as a routine, or as a program code.

The hardware used to implement the various illustrative logics, logical blocks, modules, and circuits described in connection with the aspects disclosed herein may include a general purpose processor, a digital signal processor (DSP), a special-purpose processor such as an application specific integrated circuit (ASIC) or a field programmable gate array (FPGA), a programmable logic device, discrete gate or transistor logic, discrete hardware components, or any combination thereof designed to perform the functions described herein. A general-purpose processor may be a microprocessor, but, in the alternative, the microcontroller may be any conventional processor, controller, or state machine. A microcontroller may also be implemented as a combination of computing devices, e.g., a combination of a DSP and a microprocessor, a plurality of microprocessors, one or more microprocessors in conjunction with a DSP core, or any other such configuration. Alternatively or additionally, some steps or methods may be performed by circuitry that is specific to a given function.

Many modifications and other embodiments of the inventions set forth herein will come to mind to one skilled in the art to which these inventions pertain having the benefit of teachings presented in the foregoing descriptions and the associated drawings. Although the figures only show certain components of the apparatus and systems described herein, it is understood that various other components may be used in conjunction with the system. Therefore, it is to be understood that the inventions are not to be limited to the specific embodiments disclosed and that modifications and other embodiments are intended to be included within the scope of the appended claims. Moreover, the steps in the method described above may not necessarily occur in the order depicted in the accompanying diagrams, and in some cases one or more of the steps depicted may occur substantially simultaneously, or additional steps may be involved. Although specific terms are employed herein, they are used in a generic and descriptive sense only and not for purposes of limitation.

The various illustrative logical blocks, modules, circuits, and algorithm steps described in connection with the embodiments disclosed herein may be implemented as electronic hardware, computer software, or combinations of both. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention.

## Claims

1. A device for detecting adulteration in oil, comprising:
a probe 103;
a plurality of sensors 105; and
a microcontroller 107, wherein the microcontroller 107, upon receiving plurality of sensor data from the plurality of sensors 105 while the probe 103 is in contact with the oil, is configured to:
process at least one of the plurality of sensor data to obtain one or more physical parameters corresponding to the oil; and
apply a pre-trained model on the at least one of the plurality of sensor data for detecting the adulteration in the oil in real-time based on the one or more physical parameters.

2. The device of claim 1, wherein:
the plurality of sensors 105 comprises an ultrasonic sensor, a temperature sensor, a capacitive sensor, an Infrared (IR) spectrometer sensor, a viscosity sensor, and a refractive index sensor, and
the plurality of sensor data comprises ultrasonic sensor data, temperature sensor data, capacitive sensor data, IR spectrometer sensor data, viscosity sensor data, and refractive index sensor data, and
the one or more physical parameters comprise a dielectric constant, a viscosity, absorption of IR light, and a refractive index of the oil.

3. The device of claim 1, wherein the model is trained by:
receiving first pre-stored sensor data, corresponding to a pure oil without presence of an adulterated component, from the plurality of sensors 105;
receiving second pre-stored sensor data and an actual value of an adulterated component concentration corresponding to an adulterated oil from the plurality of sensors 105;
predicting concentration of the adulterated component in the adulterated oil by comparing the one or more physical parameters of the first pre-stored sensor data and the second pre-stored sensor data; and
iteratively training the model until an error between the predicted concentration of the adulterated component and the actual value of the adulterated component concentration is below a predefined error threshold, wherein the adulterated component comprises paraffin.

4. The device of claim 2, wherein the ultrasonic sensor is configured to generate the ultrasonic sensor data comprising measurements used to determine level of the oil by:
emitting an ultrasonic signal into the oil while the probe 103 is in the contact with the oil;
calculating distance between the ultrasonic sensor and surface of the oil by measuring time taken for reception of the ultrasonic signal being reflected from the surface of the oil; and
determining the level of the oil based on the calculated distance, wherein the level of the oil is used to determine volume of the oil.

5. The device of claim 2, wherein the temperature sensor is configured to:
generate the temperature sensor data comprising temperature of the oil; and
compensate temperature-dependent variations occurring in remaining sensor data of the plurality of sensor data based on the measured temperature sensor data.

6. The device of claim 2, wherein:
the refractive index sensor is configured to generate the refractive index sensor data to detect variations in the refractive index of the oil, and
the viscosity sensor is configured to generate the viscosity sensor data by detecting changes in the viscosity of the oil due to presence of the paraffin, and
the IR spectroscopy sensor is configured to generate the Infrared (IR) spectrometer sensor data by:
detecting chemical changes in the oil by emitting Infrared (IR) light through the oil;
measuring an intensity of the emitted IR light through the oil; and
determining concentration of the paraffin based on absorption of the emitted IR light in the oil.

7. The device of claim 2, wherein the capacitive sensor is configured to generate the capacitive sensor data by:
calculating a capacitance by measuring the dielectric constant of the oil that varies due to the adulteration in the oil; and
determining presence of the adulterated component in the oil by measuring a capacitance difference between a baseline capacitance of the pure oil and the calculated capacitance of the oil under consideration, wherein the baseline capacitance is calculated by measuring the dielectric constant of the pure oil without the presence of the adulterated component.

8. The device of claim 1, further comprises:
a display unit configured to display labelled data comprising at least one of: the determined concentration of the paraffin, the temperature of the oil, and a percentage of the composition of the paraffin, wherein the microcontroller 107 is further configured to transmit the labelled data to an external device via a wireless communication protocol.

9. The device of claim 1, wherein the microcontroller 107 is further configured to:
generate an alert if the determined concentration of the paraffin exceeds a predetermined paraffin concentration threshold, and
perform re-calibration of the plurality of sensor data if the IR spectrometer sensor data indicates that the determined concentration of the paraffin exceeds the predetermined paraffin concentration threshold without a corresponding change in the viscosity of the oil.

10. A method of detecting adulteration in oil, comprising:
receiving plurality of sensor data from a plurality of sensors 105 while a probe 103 is in contact with the oil;
processing at least one of the plurality of sensor data to obtain one or more physical parameters corresponding to the oil; and
applying a pre-trained model on the at least one of the plurality of sensor data for detecting the adulteration in the oil in real-time based on one or more physical parameters.
